(19) Europäisches Patentamt
European Patent Office
Office européen des brevets

(11) Numéro de publication: **0 061 408 B1**

# (12) FASCICULE DE BREVET EUROPEEN

(45) Date de publication du fascicule du brevet: **16.01.85**

(51) Int. Cl.⁴: **C 07 F 5/02**

(21) Numéro de dépôt: **82400529.2**

(22) Date de dépôt: **24.03.82**

(54) **Nouveaux monoalcoyle boranes chiraux.**

(30) Priorité: **25.03.81 GB 8109392**

(43) Date de publication de la demande: **29.09.82 Bulletin 82/39**

(45) Mention de la délivrance du brevet: **16.01.85 Bulletin 85/3**

(84) Etats contractants désignés: **AT BE CH DE FR GB IT LI LU NL SE**

(56) Documents cités:
**US - A - 3 254 129**

**JOURNAL OF THE AMERICAN CHEMICAL SOCIETY, vol. 81, no. 1, 20 janvier 1959, H.C. BROWN et al. "A stereospecific cis hydration of the double bond in cyclic derivatives", page 247**
**JOURNAL OF ORGANOMETALLIC CHEMISTRY, vol. 156, 1978, ELSEVIER SEQUOIA S.A., (NL), A.K. MANDEL et al. "Anomalies in the asymmetric hydroboration of olefins with the 1/1 adduct of (+) alpha-pinen and BH3 - THF*", pages 183-90**

(73) Titulaire: **Société d'Expansion Scientifique EXPANSIA Société Anonyme, 264, rue du Faubourg Saint-Honoré, F-75008 Paris (FR)**

(72) Inventeur: **Aspisi, Christian, Les Grands Vallons Chemin du Breuil, F-13150 Boulbon (FR)**
Inventeur: **Bonato, Marc, Mas de la Pomparesse, F-30390 Aramon (FR)**
Inventeur: **Jacquier, Robert, 445 Avenue Valéry Larbaud, F-34000 Montpellier (FR)**

(74) Mandataire: **Lachassagne, Jacques, Boite Postale 07, F-78430 Louveciennes (FR)**

EP 0 061 408 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne les organoboranes optiquement actifs pouvant être utilisés dans des réactions d'hydroboration et de réduction asymétriques.

La plupart des agents chiraux d'hydroboration et de réduction connus présentent l'inconvénient d'être préparés *in situ*, d'être peu stables et de ne pas être solides, ce qui gêne à la fois leur commercialisation et leur manipulation. Les organoboranes chiraux obtenus selon l'invention sont solides, stables et peuvent être stockés pendant de nombreux mois à basse température.

L'invention concerne des organoboranes chiraux de formule générale (I):

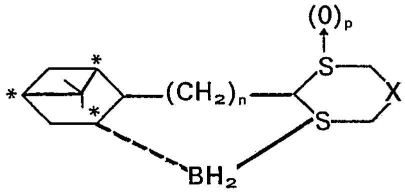

(I)

dans laquelle X représente un groupement méthylène ou un atome de soufre, n et p prenant les valeurs 0 ou 1.

Ces organoboranes chiraux sont miscibles ou solubles dans différents solvants organiques tels que le chlorure de méthylène, le benzène, l'éther éthylique, le n-pentane, le tétrahydrofuranne et le diglyme. A la température ambiante, ces composés sont solides. Les points de fusion des corps solides sont indiqués.

Leur potentialité d'induction asymétrique en réaction d'hydroboration est au moins égale à celle des autres agents d'hydroboration précédemment utilisés, tels le mono-isopinocamphéylborane, le diisopinocamphéylborane et le dilongifolyborane, mais le mono-isopinocamphéylborane et le diisopinocamphéylborane doivent être préparés *in situ* avant d'effectuer la réaction, tandis que le dilongifolyborane (P.K. Jadhav et H.C. Brown, «J. Org. Chem.», 1981, *46*, 2988) est un dialcoylborane solide obtenu à partir d'une matière première difficilement accessible: le longifolène. Les organoboranes chiraux de la présente invention sont, par contre, faciles à obtenir, solides, stables,stockables et d'un emploi aisé; ils n'ont pas nécessairement besoin d'être préparés fraîchement avant leur utilisation; ce sont des monoalcoylboranes optiquement actifs, facilement accessibles à partir de matières premières commerciales.

On peut préparer ces composés selon une méthode dérivée de celle décrite par H.C. Brown («Organic Synthesis via Boranes», Wiley Interscience, New York, NY, 1975), en utilisant un appareillage similaire.

Plus précisément, on peut obtenir ces organoboranes selon cette invention en faisant réagir, pendant 15 min à 2 h dans un solvant aprotique, sous atmosphère inerte, à une température comprise entre −20 et +40°C et, de préférence, aux alentours de 0°C, un composé de formule générale (II) (di- ou trithiane portant un substituant bicyclique) dans laquelle n, p et X sont définis comme plus haut, avec un borane tel que $B_2H_6$ ou $BH_3$/base de Lewis:

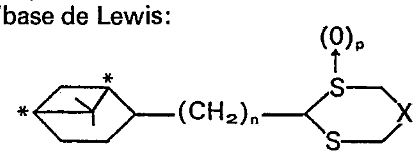

(II)

Les composés dans lesquels n=1, X=$XH_2$ ou S, p=0,1, sont obtenus à partir du (−)myrténol commercial, par halogénation selon la méthode de G. Ohloff, F. Farnow et G. Schade, «Chem. Ber.», 1956, *89*, 1549, suivie de l'échange métal/halogène entre l'organolithium du dithiane-1,3 ou du trithiane-1,3,5 (X=S) et le dérivé halogéné selon la méthode décrite par D. Seebach et E.J. Corey, «J. Org. Chem.», 1975, *40*, 231, et d'une éventuelle oxydation (p=1) par le métaperiodate de sodium selon la méthode décrite par Q.M. Carlson et P.M. Helquist, «J. Org. Chem.», 1969, *33*, 2596.

Les composés dans lesquels X=$CH_2$, n=0, p=0,1, sont obtenus par condensation du (−) myrténal et du propanedithiol-1,3 commerciaux, selon une méthode dérivée de celle décrite par P. Stutz et P.A. Stadler, «Org. Synth.», 1977, *56*, 8. Le produit de cyclisation peut être oxydé par le métaperiodate de sodium.

On peut obtenir le borane par toutes les voies traditionnelles bien connues ou à partir d'un complexe $BH_3$/base de Lewis. On peut, par exemple, obtenir le complexe de borane par la méthode décrite par H.C. Brown et A.K. Mandal, «Synthesis», 1980, 153, où le vecteur borane est le complexe $BH_3$/THF, ou par celle décrite par M. Anez, G. Uribe, L. Mendoza et R. Contrebas, «Synthesis», 1981 (3), 214, où le vecteur borane est le diborane gaz.

Les organoboranes de formule générale (I), comme défini ci-avant, sont utilisés comme agents d'hydroboration ou de réduction, respectivement.

L'invention sera d'ailleurs mieux comprise grâce à la description des exemples qui suivent.

*Exemple 1:*

*Myrtanyl-2 dithiane-1,3 borane* n=1, X=$CH_2$, p=0

125 g (0,429 mol) de myrtényl-2 dithiane-1,3 ($[\alpha]^{24}_{D}$−8,67, c=5, benzène) dissous dans 600 ml de tétrahydrofuranne anhydre sont versés dans un tricol de 1 l, maintenu sous circulation d'azote, 0,256 mol de diborane ($B_2H_6$) est injectée lentement, en 1½ h, dans le milieu réactionnel refroidi à −10°C.

Le diborane est obtenu par génération à partir de 14,25 g (0,375 mol de borohydrure de sodium (pureté 98%) dissous dans 200 ml de diglyme — préalablement distillé sur hydrure d'aluminium-lithium — sur lequel on fait couler, goutte à goutte 61,5 ml (0,5 mol) de trifluorure de bore-éthérate, également préalablement distillé. Le mélange $BH_4Na/BF_3/(C_2H_5)_2O$ a été chauffé à 60°C pendant 15 min.

Après avoir laissé le mélange réactionnel revenir à la température ambiante, on le conserve sous azote et sous agitation pendant 26 h. La fin de la réaction d'hydroboration est déterminée par la disparition du proton éthylénique en RMN du proton. Le mélange réactionnel est alors concentré sous pression réduite. On obtient un solide blanc (p.f.=90°C banc Kofler) avec un rendement de 93% ($[\alpha]_D^{24}$= −41,79, c=5, benzène).

L'identité et la structure de ce composé ont été confirmées par l'analyse comme indiqué après les exemples.

*Exemple 2:*

*Myrtanyl-2 dithiane-1,3 oxyde-1 borane* n=1, p=1, X=$CH_2$

Ce composé a été obtenu (rendement 95%) en procédant comme dans l'exemple 1, mais en utilisant du myrtényl-2 dithiane-1,3 oxyde-1 ($[\alpha]_D^{20}$ = −8, c=5,9, benzène) et du dichlorométhane comme solvant. Ce composé est un solide blanc fondant à 70-75°C (banc Kofler) ($[\alpha]_D^{21}$ = +9,12, c=5, benzène). L'identité et la structure de ce composé ont été confirmées par l'analyse comme indiqué après les exemples.

*Exemple 3:*

*Myrtanyl-2 trithiane-1,3,5 borane* n=1, p=0, X=S

Ce composé a été obtenu (rendement 94%) en procédant comme dans l'exemple 1, mais en utilisant du myrtényl-2 trithiane-1,3,5 ($[\alpha]_D^{21}$ = −12,16, c=5, benzène). Ce composé est un solide blanc fondant à 98-100°C (banc Kofler) ($[\alpha]_D^{21}$ = −4,6, c=5, benzène). L'identité et la structure de ce composé ont été confirmées par l'analyse comme indiqué après les exemples.

*Exemple 4:*

*(Diméthyl-7,7 bicyclo-[3.1.1]-heptyl)-2 dithiane-1,3 borane* n=0, p=0, X=$CH_2$

Ce composé a été obtenu (rendement 96%), en procédant comme dans l'exemple 1, mais en utilisant le (diméthyl-7,7 bicyclo-[3.1.1]-hept-2,3 ène)-2 dithiane-1,3 ($[\alpha]_D^{24}$ = −38,48, c=4,2 benzène). Ce composé est un solide blanc fondant à 64-67°C (banc Kofler) ($[\alpha]_D^{24}$= +14,02, c=4,1, benzène). L'identité et la structure de ce composé ont été confirmées par l'analyse comme indiqué après les exemples.

La stœchiométrie et la pureté des complexes obtenus dans ces exemples sont vérifiées par méthanolyse selon la méthode décrite par J. Beres, A. Dodds, A.J. Morabito et R.M. Adams, «Inorg. Chem.», 1971, *10*, 2072, et par micro-analyse. La structure est confirmée par analyse infrarouge, montrant des bandes d'absorption entre 2360 et 2400 $cm^{-1}$ (dans $CCl_4$ à 2%), caractéristiques des liaisons B−H (H.C. Brown, E. Negishi et J.J. Katz, «J. Amer. Chem. Soc.», 1975, *97*, 1791) et par résonance magnétique nucléaire ($H^1$) indiquant à la fois la disparition du proton éthylénique du composé de départ et la présence de $OCH_3$ du $-B(OCH_3)$ obtenu après méthanolyse de l'organoborane, ayant des déplacements chimiques caractéristiques à 3,50 ppm (dans 1 ml de $CCl_4$ et 0,45 ml de benzène) selon la méthode décrite par A.K. Mandal et N.M. Yoon, «J. Organometal. Chem.», 1978, *156*, 183. La structure est aussi confirmée par résonance magnétique nucléaire ($B^{11}$), indiquant un massif entre 9 et 10 ppm selon les composés (étalon interne ou externe $BF_3/(CH_2H_5)_2O$). La spectroscopie de masse indique la masse molaire des composés avec, comme pic parent, une perte d'un B−H.

La stabilité des complexes obtenus a été étudiée pendant de nombreux mois et vérifiée régulièrement par méthanolyse et par spectrométrie infrarouge. Les produits stockés sous azote et à basse température n'ont montré aucune altération.

L'activité et l'intérêt de ces composés selon l'invention ont été vérifiés en les utilisant:

A) Dans des réactions d'hydroboration par une méthode dérivée de celle décrite par A.K. Mandal et N.M. Yoon, «J. Organometal. Chem.», 1978, *156*, 183 pour le cisbutène-2, de celle décrite par P.K. Jadhav et H.C. Brown, «J. Org. Chem.», 1981, *46*, 2988 pour le méthyl-2 butène-2, et de celle décrite par H.C. Brown et N.M. Yoon, «J. Amer. Chem. Soc.», 1977, *99*, 5514 pour le méthyl-1 cyclohexène.

Par exemple, les organoboranes optiquement actifs obtenus après réaction du myrtényl-2 dithiane-1,3 borane ($MDBH_2$) sur ces alcènes sont oxydés en alcools par les méthodes classiques d'oxydation d'organoboranes (H.C. Brown, brevet US No 3254129). Les alcools obtenus ont été purifiés en chromatographie préparative gazeuse (Carbowax® 400, 20 M), afin de déterminer leur pouvoir rotatoire et de le comparer à ceux décrits dans la littérature (tableau ci-après).

*(Tableau en page suivante)*

L'hydroboration asymétrique du méthyl-2 butène-2 par le mono-isopinocamphéylborane ($IPCBH_2$) préparé par la méthode décrite par P.K. Jadhav et H.C. Brown, «J. Org. Chem.», 1981, *46*, 2988 a conduit à l'obtention du méthyl-3 butanol-2 avec une pureté optique de 9,8% (fondé sur la rotation maximale ($[\alpha]_D^{27}$ +4,86, W.A. Sanderson et M.J. Mosher, «J. Amer. Chem. Soc.», 1966, *88*, 4185), après purification en chromatographie préparative gazeuse (Carbowax® 400).

La différence de pureté optique −9,8 et 52% (citée par P.K. Jadhav et H.C. Brown, «J. Org. Chem.», 1981, *46*, 2988) rend bien compte de la difficulté de la synthèse du mono-isopinocamphéylborane; l'$IPCBH_2$ est le seul monoalkylborane décrit dans la littérature comme étant optiquement actif, non isolé, et pouvant être utilisé dans les réactions d'hydroboration asymétriques.

B) Dans des réactions de réduction asymétrique. Par exemple, la réduction asymétrique de l'acétophénone par le (diméthyl-7,7 bicyclo-[3.1.1]-heptyl)-2 dithiane-1,3 borane ($PiBH_2$) a été effectuée par une méthode dérivée de celle décrite par H.C. Brown et A.K. Mandal, «J. Org. Chem.», 1977, *42*, 2996. Le phényl-1 éthanol obtenu après oxydation de l'organoborane optiquement actif a été purifié en chromatographie prépa-

*Hydroborations asymétriques d'oléfines avec les divers organoboranes chiraux*

| Oléfines / Alcools | Cisbutène-2 / Butanol-2 | Méthyl-2 butène-2 / Méthyl-3 butanol-2 | Méthyl-1 cyclohexène / Transméthyl-2 cyclohexanol |
|---|---|---|---|
| [α] D théoriques | $[\alpha]^{25}_{D}$ − 13,5 pur[a] | $[\alpha]^{27}_{D}$ + 4,96[b] | $[\alpha]^{20}_{D}$ + 43,1[c]<br>+ 42,9<br>c: 1 $CH_3OH$ |
| (−)$IPC_2BH$ | 98,4%[d] R | 15%[d] S | —[e] |
| $(Lgf)_2BH$ | 78% [d] R | 70%[d] R | — |
| (−)$IPCBH_2$ | 25% [f] S | 52%[d] S | 72% [g] 1S 2S |
| (−)$MDBH_2$ | 27,5% R | 50% R | 67,5% 1R 2R |

$IPCBH_2$ = mono-isopinocamphéylborane
$IPC_2BH$ = diisopinocamphéylborane
$(Lgf)_2BH$ = dilongifolyborane
[a] P.J. Leroux et H.J. Lucas, «J. Amer. Chem. Soc.», 1951, *73*, 41.
[b] W.A. Sanderson et H.S. Mosher, «J. Amer. Chem. Soc.», 1966, *88*, 4185.
[c] R. Backstrom et B. Sjöberg, «Arkiv för Kemi», 1967, *26*, 549.
[d] P.K. Jadhav et H.C. Brown, «J. Org. Chem.», 1981, *46*, 2988.
[e] Pas de réactions de ces dialkylboranes sur le méthyl-2 cyclohexène. Alcène difficile pour obtenir une induction asymétrique. H.C. Brown, N.R. Ayancar et G. Zweifel, «J. Amer. Chem. Soc.», 1964, *86*, 1071.
[f] A.K. Mandal et N.M. Yoon, «J. Organometal. Chem.», 1978, *156*, 183.
[g] H.C. Brown et N.M. Yoon, «J. Amer. Chem. Soc.», 1977, *99*, 5514.

rative gazeuse (colonne Carbowax® 20 M). La pureté optique du phényl-1 éthanol est de 16,7% (fondé sur la rotation maximale trouvée dans la littérature ($[\alpha]^{23}_{D}$ = −45,5, c=5, méthanol, «Handbook of Chemistry and Physics», 1976-7, 57e édition, CRC Presse, c 297).

*Réductions asymétriques de l'acétophénone par les divers organoboranes chiraux*

| | (−)$IPC_2BH$ | (−)$IPCBH_2$ | (+)$PiBH_2$ |
|---|---|---|---|
| Acétophénonephényl-1 éthanol (%) | 9[a] | 15[b] | 16,7 |

[a] H.C. Brown et A.K. Mandal, «J. Org. Chem.», 1977, *42*, 2996.
[b] Observations non publiées. Résultats cités par H.C. Brown, P.K. Jadhav et A.K. Mandal, «Tetrahedron», 1981, *37* (21), 3547.

Les nouveaux monoalcoylboranes selon l'invention sont donc des agents d'hydroboration ou de réduction asymétrique solides, donc d'une manipulation facile. Ils sont stables et peuvent être stockés à basse température (0-4°C) plusieurs mois avant leur utilisation. Ils permettent d'effectuer des réactions de synthèse stéréosélectives et énantiosélectives.

**Revendications** pour les Etats contractants BE CH DE FR GB IT LI LU NL SE

1. Nouveaux organoboranes chiraux répondant à la formule générale:

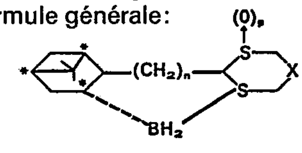

(I)

dans laquelle X représente un radical méthylène ou un atome de soufre, tandis que n et p peuvent prendre les valeurs 0 et 1.

2. Procédé de préparation des produits selon la revendication 1 consistant à faire réagir pendant une durée comprise entre 15 min et 2 h, sous atmosphère inerte, dans un solvant aprotique à une température comprise entre −20 et +40°C un composé de formule (II):

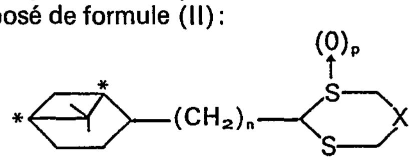

(II)

dans laquelle X, n, p sont définis comme dans la revendication 1, avec un borane de formule $B_2H_6$ ou $BH_3$/base de Lewis.

**Revendication** pour l'Etat contractant: AT

Procédé de préparation des nouveaux organoboranes chiraux répondant à la formule générale:

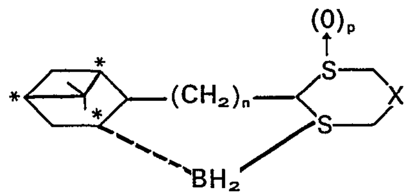

(I)

dans laquelle X représente un radical méthylène ou un atome de soufre, tandis que n et p peuvent prendre les valeurs 0 et 1, consistant à faire réagir pendant une durée comprise entre 15 min et 2 h, sous atmosphère inerte, dans un solvant aprotique à une température comprise entre −20 et +40°C un composé de formule (II):

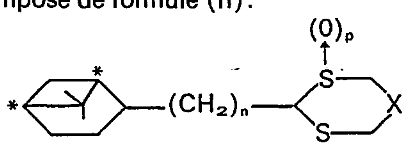

(II)

dans laquelle X, n, p sont définis comme ci-dessus, avec un borane de formule $B_2H_6$ ou $BH_3$/base de Lewis.

**Claims** for the Contracting States BE CH DE FR GB IT LI LU NL SE

1. New chiral organoboranes satisfying the general formula

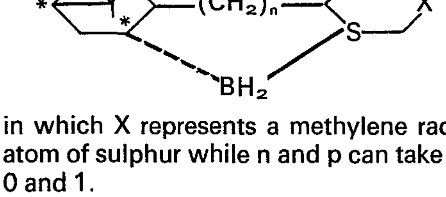

(I)

in which X represents a methylene radical or an atom of sulphur while n and p can take the values 0 and 1.

2. Process of preparation of products according to Claim 1 consisting in making react for a period comprised between 15 min and 2 h, under inert atmosphere, in an aprotic solvent at a temperature comprised between −20 and +40°C a compound of Formula II

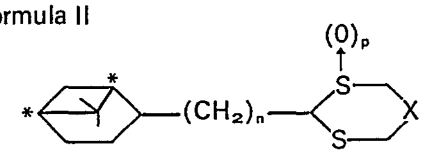

(II)

in which X, n, p are as defined in Claim 1, with a borane of formula $B_2H_6$ or $BH_3$, Lewis base.

**Claim** for the Contracting State: AT

Process of preparation of new chiral organoboranes satisfying the general formula

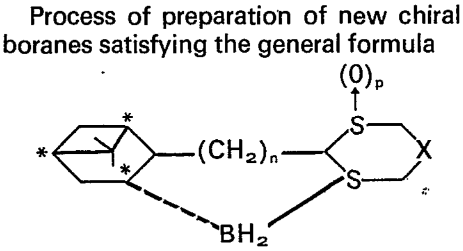

(I)

in which X represents a methylene radical or an atom of sulphur while n and p can take the values 0 and 1, consisting in making react for a period comprised between 15 min and 2 h, under inert atmosphere, in an aprotic solvent at a temperature comprised between −20 and +40°C compound of Formula (II)

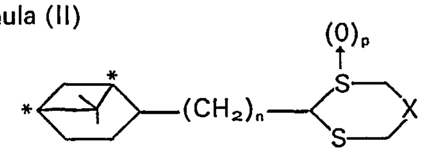

(II)

in which X, n, p are as above defined a borane of formula $B_2H_6$ or $BH_3$, Lewis base.

**Patentansprüche** für die Vertragsstaaten BE CH DE FR GB IT LI LU NL SE

1. Neue chirale organische Hydroborane der allgemeinen Formel

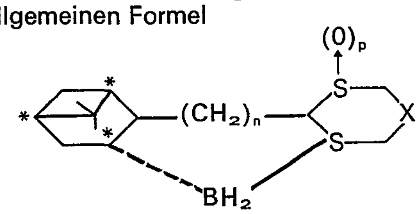

(I)

wobei X für ein Methylenradikal oder ein Schwefelatom steht und n und p die Werte 0 und 1 annehmen können.

2. Verfahren zur Herstellung der Stoffe nach Anspruch 1, bei dem während einer Zeitdauer von 15 min bis 2 h unter Inertatmosphäre und in einem aprotischen Lösungsmittel bei einer Temperatur zwischen −20 und +40°C eine Verbindung der Formel II

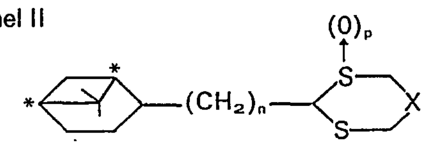

(II)

wobei X, n und p dieselbe Bedeutung haben wie

im Anspruch 1, mit einem Borhydrid der Formel $B_2H_6$ oder $BH_3$ (Lewisbase), zur Reaktion gebracht wird.

**Patentanspruch** für den Vertragsstaat: AT

Verfahren zur Herstellung neuer chiraler organischer Hydroboride der allgemeinen Formel

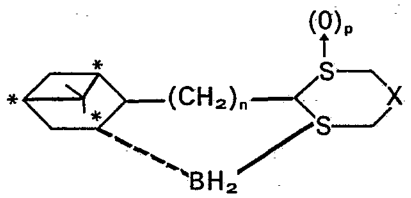

(I)

wobei X ein Methylenradikal oder ein Schwefelatom bedeutet und n und p Werte 0 oder 1 annehmen können, bei dem während einer Zeitdauer von 15 min bis 2 h unter Interatmosphäre und in einem aprotischen Lösungsmittel bei einer Temperatur zwischen −20° und +40°C eine Verbindung der Formel II

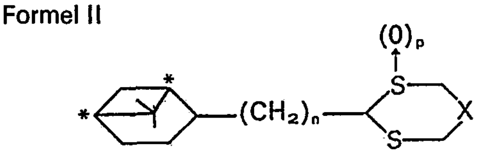

(II)

wobei X, n und p dieselbe Bedeutung haben wie in Formel (I) mit einem Borhydrid der Formel $B_2H_6$ oder $BH_3$ (Lewisbase), zur Reaktion gebracht wird.